# EUROPEAN PATENT SPECIFICATION

(11) **EP 3 083 551 B1**
(45) Date of publication and mention of the grant of the patent: **14.02.2018**
(21) Application number: 14809628.2
(22) Date of filing: 08.12.2014
(51) Int. Cl.: C07C 213/10, C07C 217/62, C07C 215/54

(54) **CRYSTALLINE FORM OF TAPENTADOL INTERMEDIATE**
KRISTALLINE FORM EINES TAPENDATOL-ZWISCHENPRODUKTS
FORME CRISTALLINE D'UN INTERMÉDIAIRE DU TAPENDATOL

(30) Priority: 16.12.2013 SI 201300430
(43) Date of publication of application: 26.10.2016
(73) Proprietor: Farma GRS, d.o.o., 8000 Novo mesto (SI)
(72) Inventor: BERGANT SIMONCIC, Ana, 8210 Trebnje (SI); KLJAJIC, Alen, 3000 Celje (SI); SMIRNOVA, Elena, 1000 Ljubljana (SI)
(74) Representative: Andrae | Westendorp Patentanwälte Partnerschaft
(86) International application number: PCT/EP2014/076916
(87) International publication number: WO 2015/091068

(56) References cited:
- EP-A1- 2 671 878
- QIANG ZHANG ET AL: "A practical and enantioselective synthesis of tapentadol", TETRAHEDRON ASYMMETRY, PERGAMON PRESS LTD, OXFORD, GB, vol. 23, no. 8, 26 March 2012 (2012-03-26) , pages 577-582, XP028517420, ISSN: 0957-4166, DOI: 10.1016/J.TETASY.2012.03.012 [retrieved on 2012-04-02] cited in the application

## Description

### FIELD OF THE INVENTION

Disclosed herein is a crystalline and stable form of (β*R*,γ*R*)-γ-ethyl-*N,N,*β-trimethyl-3-(phenylmethoxy)benzenepropanamine, as well as processes for its preparation, and further use in the preparation of tapentadol and its pharmaceutically acceptable salts.

### BACKGROUND OF THE INVENTION

Tapentadol of formula I with its chemical name 3-((1*R*,2*R*)-3-(dimethylamino)-1-ethyl-2-methylpropyl)phenol or alternatively 3-((2*R*,3*R*)-1-(dimethylamino)-2-methylpentan-3-yl)phenol, is marketed under the trade name Nucynta and indicated for the treatment of moderate to severe pain.

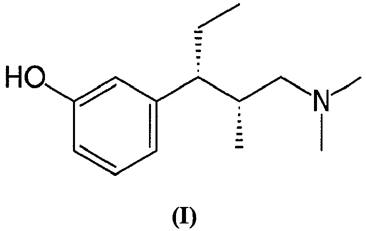

Tapentadol hydrochloride, its synthesis, and its use as an analgesic are disclosed in European patent EP0693475 B1. Various processes for the preparation of tapentadol, its enantiomers and related compounds, and their pharmaceutically acceptable salts are disclosed in EP0693475 B1; and PCT patent applications WO 2004/108658, WO 2005/000788, WO 2008/012046, WO 2008/012047, and WO 2008/012283. The basic patent for tapentadol, EP0693475 B1, discloses processes for the preparation of tapentadol or a pharmaceutically acceptable salt thereof. While it mentions that some of the disclosed compounds can form salts with physiologically acceptable acids, for example, hydrochloric acid, hydrobromic acid, sulfuric acid, methanesulfonic acid, formic acid, acetic acid, oxalic acid, succinic acid, tartaric acid, mandelic acid, fumaric acid, lactic acid, citric acid, glutamic acid and/or aspartic acid, only the hydrochloride salt of tapentadol had been prepared and isolated.

WO20121469878 discloses dimethyl compounds of formula (II)): 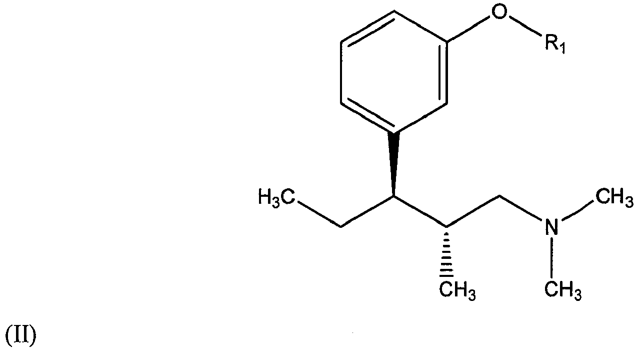 wherein R¹ represents H or a hydroxy protecting group chosen from C₁₋₆-alkyl or C₃₋₈ cycloalkyl, and its use in the preparation of tapentadol base and tapentadol pharmaceutically acceptable salts. The compound of formula (II) wherein R¹ is benzyl, is not disclosed in WO20121469878.

WO 2012103799 describes a synthesis of tapentadol via (2*R*,3*R*)-3-(3-substituted phenyl)-2-methyl-*n*-pentanamide compounds, their preparation methods and their uses in the manufacture of tapentadol or its pharmaceutically acceptable salts. Also disclosed are the intermediates in the preparation process. The compound of formula (II) wherein R¹ is benzyl, has consistently been prepared as a yellow oil and never isolated in its crystalline form; the same also applies to the article Tetrahedron: Asymmetry, Vol. 23, Issue 8, pp. 577-582 by the same authors.

It is known that synthetic compounds can contain extraneous compounds or impurities resulting from their synthesis or degradation. The impurities can be unused starting materials, by-products of the reaction, products of side reactions, or degradation products. Generally, impurities in an active pharmaceutical ingredient (API) may arise from degradation of the API itself, or during the preparation of the API. Impurities in tapentadol or any active pharmaceutical ingredient (API) are undesirable and might be harmful.

Regulatory authorities worldwide require that drug manufacturers isolate, identify and characterize the impurities in their products. Furthermore, it is required to control the levels of these impurities in the final drug compound obtained by the manufacturing process and to ensure that the impurity is present in the lowest possible levels, even if structural determination is not possible.

Because it is hard to remove some of the impurities present in the intermediates, the required purity of the final compound is difficult to achieve, leading to more crystallization steps or other separation techniques at the final stage and to much lower overall yields. It is thus of extreme importance in the case of tapentadol to remove impurities which are structurally very similar to the desired intermediate as soon as possible in the reaction scheme. Further on, particle size, for example, may affect the filterability of a drug substance. Additionally, pharmaceutical stability is believed to depend on simultaneous influence of various factors, of which some important factors include the sizes of crystals, shapes of crystals, water content, residual solvents, and impurities.

Thus, in view of the foregoing there is a need to provide (β*R*,γ*R*)-γ-ethyl-*N,N*,β-trimethyl-3-(phenylmethoxy)benzenepropanamine with improved purity, filterability as well as flowability and which also alleviates the problems associated with storing the intermediate prior to its use in the final reaction step in the preparation of tapentadol.

### SUMMARY OF THE INVENTION

This object is solved by the embodiments defined in the claims. By isolating crystalline (β*R*,γ*R*)-γ-ethyl-*N,N,*β-trimethyl-3-(phenylmethoxy)benzenepropanamine which is a -1 intermediate in the preparation of tapentadol and its pharmaceutically acceptable salts, it was surprisingly found out that persistent impurities in the final compound tapentadol and its pharmaceutically acceptable salts, in particular tapentadol hydrochloride and tapentadol maleate, could be omitted.

Aspects of the present invention provide crystals of (β*R*,γ*R*)-γ-ethyl-*N,N,*β-trimethyl-3-(phenylmethoxy)benzenepropanamine, processes for its preparation and use for the preparation of tapentadol or its pharmaceutically acceptable salts. The crystalline form of (β*R*,γ*R*)-γ-ethyl-*N,N*,β-trimethyl-3-(phenylmethoxy)benzenepropanamine enables the isolation and purification of the crude intermediate (β*R*,γ*R*)-γ-ethyl-*N,N*,β-trimethyl-3-(phenylmethoxy)benzenepropanamine with advantageously improved yields as well as an advantageously improved manufacturing process for the preparation of the final tapentadol or its pharmaceutically acceptable salts.

### BRIEF DESCRIPTION OF THE DRAWINGS

FIG. 1 is a characteristic Powder X-ray Diffraction (XRD) pattern of crystalline (β*R*,γ*R*)-γ-ethyl-*N,N*,β-trimethyl-3-(phenylmethoxy)benzenepropanamine, Form I.
FIG. 2 is a characteristic DSC thermogram of crystalline (β*R*,γ*R*)-γ-ethyl-*N,N*,β-trimethyl-3-(phenylmethoxy)benzenepropanamine, Form I.

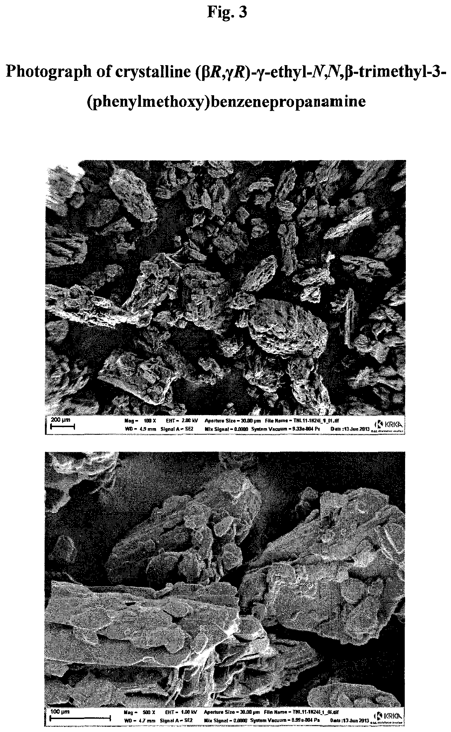

FIG. 3 is a photograph of crystalline (β*R*,γ*R*)-γ-ethyl-*N,N*,β-trimethyl-3-(phenylmethoxy)benzenepropanamine, Form I.

### DETAILED DESCRIPTION OF THE INVENTION

In particular, the present invention provides a process for preparation of crystalline (β*R*,γ*R*)-γ-ethyl-*N,N*,β-trimethyl-3-(phenylmethoxy)benzenepropanamine comprising:
(α) optionally pre-treating (β*R*,γ*R*)-γ-ethyl-*N,N,*β-trimethyl-3-(phenylmethoxy)benzenepropanamine,
   (a) providing a mixture (in particular solution, especially supersaturated solution) of (optionally pre-treated) (β*R*,γ*R*)-γ-ethyl-*N,N*,β-trimethyl-3-(phenylmethoxy)benzenepropanamine in an organic solvent or mixture thereof,
   (b) optionally, treating the mixture of step (a) with a second solvent or mixture of solvents (preferably with an appropriate anti-solvent or mixture of anti-solvents) and/or cooling, in particular to obtain supersaturated solution,
   (c) (after crystallization,) isolating material with filtration or centrifugation,
   (d) optionally, drying the isolated material, and
   (e) optionally, storing the dry, solid material (β*R*,γ*R*)-γ-ethyl-*N,N*,β-trimethyl-3-(phenylmethoxy)benzenepropanamine (preferably the dry, solid material crystalline (β*R*,γ*R*)-γ-ethyl-*N,N*,β-trimethyl-3-(phenylmethoxy)benzenepropanamine, especially the dry, solid material crystalline (β*R*,γ*R*)-γ-ethyl-*N,N*,β-trimethyl-3-(phenylmethoxy)benzenepropanamine Form I) for more than 24h.

The terms such as "about," "generally," "substantially," and the like are to be construed as modifying a term or value such that it is not an absolute. When a molecule or other material is identified herein as "substantially pure," it generally means, unless specified otherwise, that the material is at least about 95 % pure, as determined by methods that are conventional in the art such as high performance liquid chromatography (HPLC) or spectroscopic methods. "Pure" generally means, unless specified otherwise, that the material is at least about 99 % pure, as determined by such conventional methods. In general, this refers to purity with regard to unwanted residual solvents, reaction by-products, impurities, and unreacted starting materials. Percentage values are used herein to denote percent by weight, unless the context indicates otherwise.

In particular, the present invention provides a process for preparation of crystalline (β*R*,γ*R*)-γ-ethyl-*N,N,*β-trimethyl-3-(phenylmethoxy)benzenepropanamine comprising:
(α) optionally pre-treating (β*R*,γ*R*)-y-ethyl-*N,N,*β-trimethyl-3- (phenylmethoxy)benzenepropanamine,
   (a) providing a supersaturated solution of (optionally pre-treated) (β*R*,γ*R*)-γ-ethyl-*N,N*,β-trimethyl-3- (phenylmethoxy)benzenepropanamine in an organic solvent or mixture thereof,
   (b) optionally, treating the mixture (in particular supersaturated solution) of step (a) with a second solvent or mixture of solvents (preferably with an appropriate anti-solvent or mixture of anti-solvents) and/or cooling to obtain supersaturated solution,
   (c) after crystallization, isolating material with filtration or centrifugation,
   (d) optionally, drying the isolated material, and
   (e) optionally, storing the dry, solid material (β*R*,γ*R*)-γ-ethyl-*N,N,*β-trimethyl-3-(phenylmethoxy)benzenepropanamine (preferably the dry, solid material crystalline (β*R*,γ*R*)-γ-ethyl-*N*,*N*,β-trimethyl-3-(phenylmethoxy)benzenepropanamine, especially the dry, solid material crystalline (β*R*,γ*R*)-γ-ethyl-*N*,*N*,β-trimethyl-3-(phenylmethoxy)benzenepropanamine Form I) for more than 24h.

In particular, optional step (b) can be optional step (b-1) of treating the mixture (in particular solution, especially supersaturated solution) of step (a) with a second solvent or mixture of solvents (preferably with an appropriate anti-solvent or mixture of anti-solvents) (and optionally cooling) to obtain supersaturated solution, or can be optional step (b-2) of cooling the mixture (in particular solution, especially supersaturated solution) of step (a) to obtain supersaturated solution. In an embodiment, the mixture treated with optionally cooled (preferably cooled to a temperature in the second temperature range as defined below) second solvent or mixture of solvents can be cooled (preferably to a temperature in the second temperature range as defined below; in particular, the treated mixture can be cooled to a temperature of 40°C or less, e.g. below 23°C, preferably in the range from -2 to 20°C, especially in the range from -2 to 2 °C or from 15 to 20°C). The term "to obtain supersaturated solution" can in particular encompass that a supersaturated solution is obtained from a saturated or non-saturated solution, as well as that a supersaturated solution is obtained from a supersaturated solution, preferably with increased supersaturation. After step (a) or during or after step (b) crystallization can occur. The terms saturated solution and supersaturated solution are known to the skilled person and are discussed e.g. in J.W. Mullin, Crystallization, 4th ed., 2001, e.g. chapter 3.12, Butterworth-Heinemann. In the present application, a saturated solution can be in particular a solution which has the same chemical composition at a particular temperature as a solution which is in thermodynamic equilibrium with a solid phase at this particular temperature. A supersaturated solution can be in particular a solution which contains more dissolved solid than that represented by equilibrium saturation. For example, a supersaturated solution can be obtained by cooling a solution from higher temperature where the solution is saturated to a lower temperature, where the solution is supersaturated.

The mixture (in particular solution, especially supersaturated solution) provided (in step a)) can be obtained by preparing a solution by dissolving (optionally pre-treated) (β*R*,γ*R*)-γ-ethyl-*N*,*N*,β-trimethyl-3-(phenylmethoxy)benzenepropanamine in an organic solvent or mixture thereof in a first temperature range, such as e.g. above 28°C, in particular from 30°C to 65°C, e.g. 30 to 35°C. By lowering the temperature of this solution prepared by dissolving in the first temperature range and/or by removing solvent(s) (e.g. by distillation) from this solution, this solution may become a supersaturated solution or the supersaturation of this solution may be increased. The treated and/or cooled mixture (especially supersaturated solution) obtained by carrying out step (b), especially step (b-1) or (b-2), can have a temperature in a second temperature range (which is below (e.g. at least 10°C below) the first temperature range), in particular a temperature of 40°C or less, e.g. below 23°C, preferably a temperature in the range from -2 to 20 °C, especially in the range from -2 to 2 °C or from 15 to 20°C. The treated and/or cooled mixture (especially supersaturated solution) obtained by carrying out step (b), especially step (b-1) or (b-2), or the mixture (especially solution, in particular supersaturated solution) obtained by carrying out step (a) can subsequently continue to crystallize or can crystallize. In particular, a step of crystallizing the mixture or solution obtained from step (a) or (b) at a crystallization temperature can be carried out, optionally under stirring. Crystallization temperature can be 40°C or less, e.g. below 23°C, preferably below 20°C, e.g. in the range from -20 to 20 °C, especially in the range from -2 to 2 °C or from 15 to 20°C. This step of crystallizing can be carried out for a crystallization time period which can be e.g. a time period of at least 0,1 minutes, in particular of at least 1 minute, e.g. from 30 minutes to 5 days.

The organic solvent or mixture thereof set forth in step (a) is herein also referred to as first solvent or mixture thereof. First solvent or mixture thereof and second solvent or mixture of solvents can be different one from another. Preferably, the mixture of step (a) can be a solution, preferably a supersaturated solution. The term supersaturated solution can in particular refer to a solution which is supersaturated at least at the temperature or in the range of temperatures where crystallization is carried out. In particular, the supersaturated solution can be a solution which is saturated at the boiling point temperature of the organic solvent or mixture thereof or less, in particular at a temperature in the range from boiling point to 15°C below boiling point. Especially, the supersaturated solution can be a solution which is supersaturated at crystallization temperature, especially which has equilibrium saturation at least 2°C, in particular at least 5° C above crystallization temperature. Preferably, the second solvent or mixture of solvents can be an anti-solvent or mixture of anti-solvents.

The mixture of (β*R*,γ*R*)-γ-ethyl-*N*,*N*,β-trimethyl-3-(phenylmethoxy)benzenepropanamine in step (a) may be obtained by dissolving (β*R*,γ*R*)-γ-ethyl-*N*,*N*,β-trimethyl-3-(phenylmethoxy)benzenepropanamine in an organic solvent or mixture thereof, preferably in a alcohol, ester, ionic liquid, silicone, ether or nitrile, most preferably in one of acetonitrile, methanol, 1-ethyl-3-methylimidazolium ethyl sulfate, 1-ethyl-3-methylimidazolium methyl sulfate, 1-butyl-3-methylimidazolium tetrafluoroborate, ethylene glycol (ethane-1,2-diol), propylene glycol, polydimethylsiloxane, and mixtures thereof (in particular mixture comprising or consisting of ethylene glycol, and polydimethylsiloxane).

The term "an organic solvent or mixture thereof" (also referred to herein as "a first solvent or mixture thereof") can have in particular the meaning of an organic solvent or of a solvent mixture comprising or consisting of this organic solvent and at least one further solvent. Preferably, the organic solvent can be selected from the group consisting of alcohol, ester, ionic liquid, silicone, ether, and nitrile. The at least one further solvent can be in particular at least one further organic solvent, e.g. selected from the group consisting of alcohol, ester, ionic liquid, silicone, ether, and nitrile.

The alcohol used as an organic solvent or in a mixture thereof in step a) can be in particular an alkyl alcohol, which can comprise at least one (e.g. 1, 2, or 3) OH-group(s), especially a (e.g. straight chain or branched and/or cyclic) C1-C6 alkyl alcohol. The alcohol used as an organic solvent or in a mixture thereof in step a) can be in particular chosen from alkane(mono)ols, alkanediols, alkanetriols, cycloalkane(mono)ols, cycloalkanediols, cycloalkanetriols, and mixtures thereof. An alkane(mono)ol can be in particular chosen from C1-C6 alkane(mono)ols, such as methanol, ethanol, propanol (e.g. 1- or 2-propanol and mixtures thereof), butanol (e.g. n-, iso-, sec-, tert-butanol and mixtures thereof), pentanol (e.g. 1-pentanol, and all further structural isomers and mixtures thereof), hexanol (e.g. 1-hexanol, and all further structural isomers and mixtures thereof), and mixtures thereof. An alkanediol (in particular a C1-C6 alkanediol) can be a vicinal alkanediol, in particular a 1,2-alkanediol or glycol, preferably ethylene glycol or propylene glycol. An alkanediol may be in particular chosen from HO-C(H)(R^{a})-C(H)(OH)-R^{b}, wherein R^{a} is C1-C3 alkyl or H, especially one of H, methyl, and ethyl, and wherein R^{b} is C1-C3 alkyl or H, especially one of H, methyl, and ethyl. In the context of the present invention, a vicinal diole can be in particular any compound which comprises two OH-groups bonded to two adjacent carbon atoms. Particularly advantageous results were obtained for example for methanol, or mixtures comprising methanol, or for ethylene glycol, or propylene glycol, or mixtures comprising ethylene glycol or propylene glycol, as an organic solvent or mixture thereof in process step a).

The nitrile used as an organic solvent or in a mixture thereof in step a) can be in particular an alkyl nitrile (which may be e.g. straight chain or branched), especially a C1-C5 alkyl nitrile, further especially an alkyl nitrile, Rⁿ-CN, wherein Rⁿ is a C1-C4 alkyl, especially one selected from the group consisting of methyl, ethyl, propyl, and butyl.
The ester used as an organic solvent or in a mixture thereof in step a) can be in particular an alkyl ester, especially an ester R^{es}-C(O)-O-R^{es'}, wherein R^{es} can be a C1-C6 alkyl (which may be e.g. straight chain or branched), especially one selected from the group consisting of methyl, ethyl, propyl, butyl, pentyl, hexyl, and wherein R^{es'} can be a C1-C6 alkyl (which may be e.g. straight chain or branched), especially one selected from the group consisting of methyl, ethyl, propyl, butyl, pentyl, hexyl.
The ionic liquid used as an organic solvent or in a mixture thereof in step a) can be in particular chosen from 1-alkyl-3-alkylimidazolium salts, especially chosen from 1-alkyl-3-methylimidazolium salts, further especially chosen from 1-ethyl-3-methylimidazolium salts, such as 1-ethyl-3-methylimidazolium ethyl sulfate and 1-ethyl-3-methylimidazolium methyl sulfate, and 1-butyl-3-methylimidazolium salts, such as 1-butyl-3-methylimidazolium tetrafluoroborate. Preferably, 1-alkyl-3-alkylimidazolium salts may be compounds of the following formula: 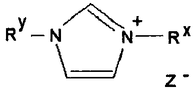 wherein R^{x} can be C1 to C6 alkyl, especially can be selected from the group consisting of methyl, ethyl, propyl, butyl, pentyl, hexyl, and RY can be C1 to C6 alkyl, especially can be selected from the group consisting of methyl, ethyl, propyl, butyl, pentyl, hexyl, preferably methyl, and Z⁻ can be an anion, in particular an organic or inorganic anion, especially an anion selected from tetrafluoroborate anion (BF₄⁻); alkylsulfate anion, such as ethylsulfate anion, methylsulfate anion; and halogenide anions. The ionic liquid can be in particular an ionic liquid which is liquid at 40°C (especially 40 °C and 101325 Pa), preferably at room temperature (e.g. 25 °C, especially 25 °C and 101325 Pa).

A silicone can be preferably a silicone which is liquid at 40°C (especially 40 °C and 101325 Pa), preferably at room temperature (e.g. 25 °C, especially 25 °C and 101325 Pa), and/or may have a boiling point of at least 180 °C, preferably a boiling point in the range from 190°C to 220°C, more preferably in the range from 195°C to 205°C, more preferably in the range from 198 to 202°C, especially of 200°C. Boiling points may be in particular determined at 1,013 hPa. Silicones which are liquid at 25°C are herein also referred to as silicone oil. Furthermore, the silicone or silicone oil can be a polydialkylsiloxane, especially a polydimethylsiloxane, which preferably can be characterized by the above-indicated boiling point and/or melting point ranges. In the context of the present application, a polydialkylsiloxane can be a silicone which comprises the structural element -(O-Si(R^{s1})(R^{s2})-)ₙ , preferably -(O-Si(CH₃)(CH₃)-)ₙ, with n being at least 1, preferably at least 2, further preferably at least 3, further preferably at least 4. Furthermore, n can be e.g. not more than 500, especially not more than 400, further especially not more than 200, further especially not more than 70, further especially not more than 10. R^{s1}, and R^{s2} can be alkyl, and can be in particular independently selected from methyl, ethyl and propyl. In particular, a polydimethylsiloxane can be of formula Q-(O-Si(CH₃)(CH₃)-)ₙO-P (Formula F-1), more preferably of formula (CH₃)₃Si-(O-Si(CH₃)(CH₃)-)ₙO-Si(CH₃)₃ (Formula F-2), wherein Q- and P- can be end groups, and n being as defined above. Endgroups are known to the skilled person. Furthermore, the silicone, especially the polydialkylsiloxane (in particular polydimethylsiloxane, preferably of formula F-1, or F-2, as shown supra), can have a dynamic viscosity of 2 to 30 mPa.s, preferably of 2.5 to 10 mPa.s, more preferably of 2.8 to 5 mPa.s, more preferably of 2.9 to 4 mPa.s, more preferably of 3 to 3.1 mPa.s, especially of 3 mPa.s. In an embodiment, the dynamic viscosity can be determined at 20°C (especially 20°C, 101325 Pa). Methods for determining the dynamic viscosity are known to the skilled person; preferably, a method according to DIN 53019 may be applied. Furthermore, the silicone, especially the polydialkylsiloxane (in particular polydimethylsiloxane), can have a density of 0.80 to 1.05 g/cm³, preferably of 0.85 to 1.00 g/cm³, more preferably of 0.88 to 0.94 g/cm³, more preferably of 0.89 to 0.90 g/cm³, especially of 0.894 g/cm³. In an embodiment, the density can be determined at 20°C (especially 20°C, 101325 Pa). Methods for determining the density are known to the skilled person; preferably, a method according to DIN 51757 may be applied. As regards DIN regulations cited herein, preferably the DIN regulation in force on the priority date of the present application should be applied.

Preferably, a solvent mixture used in step a) (which may comprise an organic solvent and at least one further solvent) may comprise at least 0.1 wt.-%, especially between 0.1 to 99.9 wt.-%, further especially at least 90 wt.-%, further especially at least 99 wt.-%, of the organic solvent, preferably selected from the group consisting of alcohol, ester, ionic liquid, silicone (in particular silicone oil), ether and nitrile, based on the total weight of the solvent mixture.

Prior to step a) optionally a step α) of pre-treating (β*R*,γ*R*)-γ-ethyl-*N*,*N*,β-trimethyl-3-(phenylmethoxy)benzenepropanamine can be carried out. This pre-treating can be in particular for obtaining (β*R*,γ*R*)-γ-ethyl-*N*,*N*,β-trimethyl-3-(phenylmethoxy)benzenepropanamine in non-crystalline form, preferably in fluid form or in non-solid form, especially in oil or solution form.

In particular, step α) can be pre-treating (β*R*,γ*R*)-γ-ethyl-*N*,*N*,β-trimethyl-3-(phenylmethoxy)benzenepropanamine with a pre-treatment solvent, especially for obtaining this compound in non-crystalline form, preferably in fluid form or in non-solid form. The pre-treatment solvent can be any solvent or mixture of solvents, wherein (β*R*,γ*R*)-γ-ethyl-*N*,*N*,β-trimethyl-3-(phenylmethoxy)benzenepropanamine may be dissolved and can be e.g. a C1 to C6 monochloralkane, a C1 to C6 dichloralkane, a C1 to C6 trichloralkane, a C1 to C6 tetrachloralkane, or mixtures thereof, preferably dichloromethane or a mixture comprising dichloromethane. According to one embodiment, the pre-treatment can comprise dissolving (β*R*,γ*R*)-γ-ethyl-*N*,*N*,β-trimethyl-3-(phenylmethoxy)benzenepropanamine in a pre-treatment solvent (such as dichloromethane), and subsequently partially or completely removing the pre-treatment solvent, especially removing the pre-treatment solvent in such an amount that the obtained pre-treated material comprises or preferably consists of (β*R*,γ*R*)-γ-ethyl-*N*,*N*,β-trimethyl-3-(phenylmethoxy)benzenepropanamine in non-crystalline form, preferably in fluid form or in non-solid form. The removal of the pre-treatment solvent may be in particular obtained by evaporating the solvent, preferably under reduced pressure (e.g. at a pressure below ambient pressure) and/or by heating. The pre-treated material (obtained after step α)) comprising or consisting of (β*R*,γ*R*)-γ-ethyl-*N,N*,β-trimethyl-3-(phenylmethoxy)benzenepropanamine in non-crystalline form may be subsequently combined and mixed with an organic solvent or mixture thereof, in particular for providing the mixture of process step a). Preferably, the pre-treated material (obtained after step α)) may contain less than 1 wt.-%, preferably less than 0.3 wt.-%, more preferably less than 0.1 wt.-% of pre-treatment solvent, based on the weight of the pre-treated material.
According to another embodiment, the pre-treatment can comprise melting (β*R*,γ*R*)-γ-ethyl-*N*,*N*,β-trimethyl-3-(phenylmethoxy)benzenepropanamine, preferably contained in a suspension containing a ionic liquid (in particular a 1-alkyl-3-alkylimidazolium salt) or a mixture thereof, or an alkanediol (in particular ethylene glycol or propylene glycol) or a mixture thereof (in particular in a mixture comprising ethylene glycol and polydimethylsiloxane).
After pre-treatment, the obtained pre-treated material can be either (β*R*,γ*R*)-γ-ethyl-*N*,*N*,β-trimethyl-3-(phenylmethoxy)benzenepropanamine in non-crystalline form, preferably in fluid form or non-solid form, or a solution comprising (β*R*,γ*R*)-γ-ethyl-*N*,*N*,β-trimethyl-3-(phenylmethoxy)benzenepropanamine and pre-treatment solvent. This solution comprising pre-treatment solvent can comprise preferably at least 97 % by weight, preferably at least 99 % by weight, more preferably at least 99,8 % by weight of (β*R*,γR)-γ-ethyl-*N*,*N*,β-trimethyl-3-(phenylmethoxy)benzenepropanamine, based on the total weight of the solution comprising pre-treatment solvent.

Highly advantageous crystallisation process results are in particular obtained by carrying out a step α) of pre-treating with a pre-treatment solvent (especially dichloromethane) in combination with a step a), in which step a) the organic solvent or mixture thereof is one of a ionic liquid (in particular a 1-alkyl-3-alkylimidazolium salt) or mixture thereof, an alcohol (in particular alkanediol (which can be branched or straight chain), such as ethylene glycol or propylene glycol) or mixture thereof, and an silicone (in particular polydialkylsiloxane, such as polydimethylsiloxane), or mixture thereof. In particular, when carrying out step α) of pre-treating with a pre-treatment solvent (such as dichloromethane), in step a) the organic solvent or mixture thereof can be selected from the group consisting of 1-ethyl-3-methylimidazolium salts, such as 1-ethyl-3-methylimidazolium ethyl sulfate and 1-ethyl-3-methylimidazolium methyl sulfate, and 1-butyl-3-methylimidazolium salts, such as 1-butyl-3-methylimidazolium tetrafluoroborate, 1,2-alkanediols, in particular ethylene glycol, or propylene glycol, polydialkylsiloxanes, in particular polydimethylsiloxane, and mixtures thereof.

Furthermore, in embodiments, wherein in step a) a ionic liquid (in particular a 1-alkyl-3-alkylimidazolium salt) is used as a solvent, the (β*R*,γR)-γ-ethyl-*N,N,*β-trimethyl-3-(phenylmethoxy)benzenepropanamine may melt first and may get then dissolved into the mixture from its (partially or completely) melted state.

Alternatively, the mixture comprising (β*R*,γ*R*)-γ-ethyl-*N*,*N*,β-trimethyl-3-(phenylmethoxy)benzenepropanamine (in particular the mixture of step a)) may be obtained directly from a reaction in which (β*R*,γ*R*)-γ-ethyl-*N*,*N*,β-trimethyl-3-(phenylmethoxy)benzenepropanamine is formed. The mixture can be heated up to reflux temperature as long as the stability of (β*R*,γ*R*)-γ-ethyl-*N*,*N*,β-trimethyl-3-(phenylmethoxy)benzenepropanamine is not compromised and a clear solution is obtained. The time period can be as long as required for a complete dissolution. The solution can optionally be filtered by passing through paper, glass fiber, or other membrane material, or a bed of a clarifying agent such as celite. Depending upon the equipment used and the concentration and temperature of the solution, the filtration apparatus may need to be heated to avoid premature crystallization.

The mixture (in particular the mixture of step a) or a mixture obtained in step b)) may first be heated to reflux temperature for a suitable time followed by cooling and stirring for solid formation. Suitable times for crystallization will vary and can be from about 1 min to about 5 days. Suitable temperatures for crystallization are limited only by the freezing and the boiling points of solvent or solvent mixture used. Alternately, stepwise cooling can be done to ease the filtration by improving the morphology of crystalline particles. The exact temperatures and time required for complete solid formation can be readily determined by a person skilled in the art. The methods by which the solid material is recovered from the final mixture, with or without cooling below the operating temperature, can be any of techniques such as decantation, filtration by gravity or suction, centrifugation, and the like. The isolated crystals may carry a small proportion of occluded mother liquor containing a higher percentage of impurities. In particular, the isolated material isolated in step (c) can comprise crystalline (β*R*,γ*R*)-γ-ethyl-*N*,*N*,β-trimethyl-3-(phenylmethoxy)benzenepropanamine, especially Form I thereof. Furthermore, the isolated material isolated in step (c) can comprise organic solvent or mixture thereof and/or second solvent or mixture of solvents (in particular antisolvent or mixture of antisolvents). If desired, the isolated crystals may be washed with a solvent to wash out the mother liquor.

The wet cake obtained by above processes may optionally be further dried. Drying (in particular step d) of drying the isolated material) can be suitably carried out in a tray dryer, vacuum oven, air oven, fluidized bed dryer, spin flash dryer, flash dryer and the like. The drying can be carried out at temperatures of about 10 to about 41 °C, with or without applying lower pressure. The drying can be carried out for any desired times until the desired product purity is achieved. The isolated crystalline (β*R*,γ*R*)-γ-ethyl-*N,N,*β-trimethyl-3-(phenylmethoxy)benzenepropanamine can be present in any form which include but not limited to the anhydrate, a solvate, or a hydrate.

According to an embodiment, the process for preparation of crystalline (β*R*,γ*R*)-γ-ethyl-*N,N*,β-trimethyl-3-(phenylmethoxy)benzenepropanamine comprises step (b) as a non-optional step, in particular steps (b) and (d) as non-optional steps, especially steps (b), (d) and (e) as non-optional steps. According to another embodiment, the process for preparation of crystalline (β*R*,γ*R*)-γ-ethyl-*N,N*,β-trimethyl-3-(phenylmethoxy)benzenepropanamine comprises step (d) as a non-optional step, in particular steps (d) and (e) as non-optional steps. According to another embodiment, the process for preparation of crystalline (β*R*,γ*R*)-γ-ethyl-*N,N*,β-trimethyl-3-(phenylmethoxy)benzenepropanamine comprises step (α) as a non-optional step, in particular steps (α) and (d) as non-optional steps.

According to an embodiment, the material obtained after step (d) can be crystalline (β*R*,γ*R*)-γ-ethyl-*N,N*,β-trimethyl-3-(phenylmethoxy)benzenepropanamine (especially Form I thereof) or can comprise this crystalline compound (especially Form I thereof), in particular can comprise at least 97 wt.%, especially at least 99 wt.%, further especially at least 99,5 wt.% of crystalline Form I thereof, based on the total weight of the material obtained after step (d).

The (β*R*,γ*R*)-γ-ethyl-*N,N,*β-trimethyl-3-(phenylmethoxy)benzenepropanamine (which can be obtainable by a process according to the present invention) is substantially free from impurities. Typically, the (β*R*,γ*R*)-γ-ethyl-*N,N,*β-trimethyl-3-(phenylmethoxy)benzenepropanamine is of high purity, such as at least about 95 %, in particular at least about 97 %, at least about 99 %, at least about 99.5 %, or at least about 99.9 %, by weight pure. Correspondingly, the level of impurities may be less than about 1 %, less than 0.5 %, or less than 0.1 %, by weight, as determined using high performance liquid chromatography (HPLC).

The second solvent or mixture of solvents can be an antisolvent or mixture of antisolvents. An antisolvent or mixture of antisolvents can be in particular any fluid, preferably solvent, which can decrease the solubility of (β*R*,γR)-γ-ethyl-*N*,*N*,β-trimethyl-3-(phenylmethoxy)benzenepropanamine present in the mixture of (β*R*,γ*R*)-γ-ethyl-*N,N*,β-trimethyl-3-(phenylmethoxy)benzenepropanamine in an organic solvent or mixture thereof and/or can enhance the precipitation of (β*R*,γ*R*)-γ-ethyl-*N,N,*β-trimethyl-3-(phenylmethoxy)benzenepropanamine from the mixture of (β*R*,γ*R*)-γ-ethyl-*N,N*,β-trimethyl-3-(phenylmethoxy)benzenepropanamine in an organic solvent or mixture thereof. A preferred second solvent, in particular antisolvent, can be a fluid (in particular solvent) which can be polar, in particular can be more polar than the organic solvent used in step a) or can be more polar than at least one, preferably each of the organic solvent mixture components used in step a). A preferred second mixture of solvents, in particular a preferred mixture of antisolvents, can be a mixture consisting of fluids (in particular solvents), wherein the mixture component present in the highest weight amount in the second mixture of solvents, in particular mixture of antisolvents, is more polar than the organic solvent used in step a) or is more polar than at least one, preferably each of the organic solvent mixture components used in step a). Preferably all mixture components of the second mixture of solvents, in particular mixture of antisolvents, are more polar than the organic solvent used in step a) or than each of the organic solvent mixture components used in step a). In one embodiment, the polarity of a compound (solvent) can be determined according to Y, or Z or E_{T} polarity scale, preferably E_{T} polarity scale. These scales are known to the skilled person and are e.g. described in J. March, Advanced Organic Chemistry, 3rd ed., 1985, John Wiley and Sons, Inc., p. 318-320 and in the references cited therein. The second solvent or mixture of solvents, in particular anti-solvent or mixture of anti-solvents, of step b) can be a compound or a mixture of compounds different from the organic solvent or mixture thereof of step a). If a second mixture of solvents, in particular mixture of anti-solvents, of step b) consists in some embodiments of the same components as the organic solvent mixture of step a), said second mixture of solvents, in particular mixture of anti-solvents of step b) will at least contain at least one of these components in different weight amount. The term fluid can in particular refer to a fluid which is in fluid form, preferably liquid, at room temperature (e.g. 25°C, especially 25°C and 101325 Pa).

In particular, a second solvent or mixture of solvents, preferably an antisolvent or mixture of antisolvents, can be a compound or mixture of compounds (especially any solvent or mixture of solvents) which can have a relative permittivity (also called dielectric constant) of more than 20, preferably of more than 25, preferably of more than 35. Furthermore, an organic solvent or mixture thereof can be in several embodiments a solvent or mixture of solvents which can have a relative permittivity (also called dielectric constant) of less than 20, preferably of less than 19. Relative permittivity (also called dielectric constant) may be defined as the dimensionless ratio of the permittivity of the dielectric to the permittivity of a vacuum. It may be determined by obtaining the ratio of the capacitance of a capacitator completely filled with the dielectric to the capacitance of the capacitator when evacuated (International Union of Pure and Applied Chemistry, Physical Chemistry Division, Commission on Physicochemical Measurements and Standards, Pure & Appl. Chem., Vol. 53, pp. 1847-1862). Relative permittivity values may refer to solvent at a temperature of 293.15 K and a pressure of 10⁵ Pa. In particular, a second solvent or mixture of solvents, preferably an antisolvent or mixture of antisolvents, can have a relative permittivity preferably at least 1.2 times, more preferably at least 1.5, more preferably at least 2 times, especially at least 2.5 times higher than the relative permittivity of the organic solvent or mixture thereof used in step a).
In particular, in step (a) a mixture of (β*R*,γ*R*)-γ-ethyl-*N,N*,β-trimethyl-3-(phenylmethoxy)benzenepropanamine (abbreviated in the following also as (β*R*,γ*R*)-A) in an organic solvent or mixture thereof, can be provided, and in step (b) the (β*R*,γ*R*)-A containing mixture of step (a) can be treated with a second solvent (in particular an antisolvent), which is a compound having a relative permittivity which is higher (preferably at least 1.2 times, more preferably at least 1.5, more preferably at least 2 times, especially at least 2.5 times higher) than the relative permittivity of the organic solvent or mixture thereof used in step a) or can be treated with a second mixture of solvents (in particular mixture of anti-solvents), which is a mixture having a relative permittivity which is higher (preferably at least 1.2 times, more preferably at least 1.5 times, more preferably at least 2 times, especially at least 2.5 times higher) than the relative permittivity of the organic solvent or mixture thereof used in step a). In particular, all components of the second mixture of solvents (in particular mixture of anti-solvents) can have a relative permittivity which is higher (preferably at least 1.2 times, more preferably at least 1.5 times, more preferably at least 2 times, especially at least 2.5 times higher) than the relative permittivity of the organic solvent or mixture thereof used in step a), preferably higher than the relative permittivity of all components of the organic solvent mixture used in step a).

In the context of the present application, the term "mixture of anti-solvents" may refer to a mixture comprising at least one anti-solvent, especially may refer to a mixture comprising two or more anti-solvents, and in particular may refer to a mixture consisting of anti-solvents.

Preferably, a second solvent or mixture of solvents (in particular an antisolvent or mixture of antisolvents) can be selected from the group consisting of water, alkanediols, solvent mixtures comprising water, and solvent mixtures comprising alkanediols. An alkanediol (in particular a C1-C6 alkanediol) can be a vicinal alkanediol, in particular a 1,2-alkanediol or glycol, preferably ethylene glycol or propylene glycol. An alkanediol may be in particular chosen from HO-C(H)(R^{a})-C(H)(OH)-R^{b}, wherein R^{a} is C1 to C3 alkyl or H, especially one of H, methyl, and ethyl, and wherein R^{b} is C1 to C3 alkyl or H, especially one of H, methyl, and ethyl.

In step (a) a mixture of (β*R*,γ*R*)-γ-ethyl-*N,N*,β-trimethyl-3-(phenylmethoxy)benzenepropanamine in an alcohol, preferably alkane(mono)ol, preferably methanol, or in a mixture comprising alcohol, preferably comprising alkane(mono)ol, preferably comprising methanol, can be provided, and in step (b) the mixture of step (a) can be treated with an antisolvent, which is water, or a solvent mixture comprising water, or with a mixture of antisolvents, which can be a mixture of one or more antisolvents and/or water. The alkane(mono)ol can be in particular chosen from C1-C6 alkane(mono)ols, such as methanol, ethanol, propanol (e.g. 1- or 2-propanol and mixtures thereof), butanol (e.g. n-, iso-, sec-, tert-butanol and mixtures thereof), pentanol (e.g. 1-pentanol, and all further structural isomers and mixtures thereof), hexanol (e.g. 1-hexanol, and all further structural isomers and mixtures thereof), and mixtures thereof, preferably methanol.

Still furthermore, in step (a) a mixture of (β*R*,γ*R*)-γ-ethyl-*N,N*,β-trimethyl-3-(phenylmethoxy)benzenepropanamine in a nitrile, preferably in an alkylnitrile, more preferably in acetonitrile, or in a mixture comprising a nitrile, preferably an alkylnitrile, more preferably acetonitrile, can be provided, and in step (b) the mixture of step (a) can be treated with an antisolvent, which is water, or a solvent mixture comprising water, or with a mixture of antisolvents, which can be a mixture of one or more antisolvents and water.

Step (e) of storing the dry, solid material (β*R*,γ*R*)-γ-ethyl-*N,N*,β-trimethyl-3-(phenylmethoxy)-benzenepropanamine can be carried out for more than 24 hours (h), preferably for a period from 25 h to 10 years. Crystalline (β*R*,γ*R*)-γ-ethyl-*N,N*,β-trimethyl-3-(phenylmethoxy)benzenepropanamine, in particular Form I thereof, can be obtained by carrying out steps (a) to (d), especially steps (a) to (e) of the process of the present invention. The storing can be carried out for example in the temperature range of up to 100°C, in particular of up to the melting temperature of Form I, preferably in the temperature range between -40°C to 40°C, more preferably in the temperature range from about 2°C to about 8°C.

Furthermore, the present inventors provide a process for the preparation of tapentadol or a pharmaceutically acceptable salt thereof, especially a process for the preparation of (β*R*,γ*R*)-γ-ethyl-*N,N*,β-trimethyl-3-(phenylmethoxy)benzenepropanamine and for the subsequent preparation of tapentadol or a pharmaceutically acceptable salt thereof, which comprises:
(f) preparing tapentadol or a pharmaceutically acceptable salt thereof from the material isolated with filtration or centrifugation (which can be obtained in particular by step (c) of isolating material with filtration or centrifugation), or from the dried isolated material (which can be obtained in particular from step (d) of drying the isolated material), or from the stored (β*R*,γ*R*)-γ-ethyl-*N,N*,β-trimethyl-3-(phenylmethoxy)benzenepropanamine, which can be obtained by step e) of storing the dry, solid material (β*R*,γ*R*)-γ-ethyl-*N,N*,β-trimethyl-3-(phenylmethoxy)benzenepropanamine for more than 24 h. Dry, solid material (β*R*,γ*R*)-γ-ethyl-*N,N*,β-trimethyl-3-(phenylmethoxy)benzenepropanamine, in particular crystalline Form I thereof, can be obtained by carrying out process steps (a) to (d) discussed supra. Step (f) may be carried out subsequently to step (e) discussed supra, or subsequently to process step (d) discussed supra, or subsequently to process step (c) supra. In particular, stored (β*R*,γ*R*)-γ-ethyl-*N,N*,β-trimethyl-3-(phenylmethoxy)benzenepropanamine can be crystalline, especially Form I thereof.

According to another aspect, the present invention pertains to the use of crystalline (β*R*,γ*R*)-γ-ethyl-*N,N*,β-trimethyl-3-(phenylmethoxy)benzenepropanamine for the preparation of tapentadol or a pharmaceutically acceptable salt thereof.

(β*R*,γ*R*)-γ-ethyl-*N,N*,β-trimethyl-3-(phenylmethoxy)benzenepropanamine isolated by the procedure and in crystalline form as according to this invention may be prepared according to any process known or anticipated from prior art. It may further be used to prepare tapentadol free base (in solid form, oily form or solution, in particular solution in the specified solvents, in a reaction mixture or in a diluted reaction mixture) according to any of the reported manufacturing method, or any method employed by the person skilled in the art. Tapentadol thus prepared may be isolated in its base form or in the form of any pharmaceutically acceptable salt. A suitable method comprises deprotection of the *O-*protected intermediate (β*R*,γ*R*)-γ-ethyl-*N,N*,β-trimethyl-3-(phenylmethoxy)benzenepropanamine, according to the invention, in salt form and conversion of the tapentadol salt thus obtained into the free base form. Another suitable method comprises deprotection of an *O*-protected intermediate, such as (β*R*,γ*R*)-γ-ethyl-*N,N*,β-trimethyl-3-(phenylmethoxy)benzenepropanamine, in the free base form.

In case tapentadol base of increased purity is desired, the preparation of tapentadol maleate may also be used as a purification step, and the tapentadol maleate then converted to tapentadol base or another tapentadol salt.

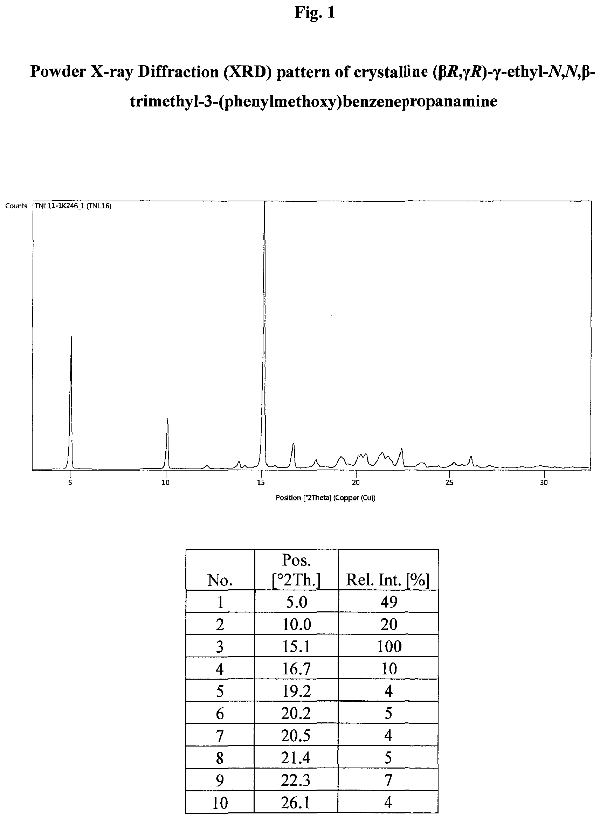

Another aspect of the present invention is the polymorphic form of the (β*R*,γ*R*)-γ-ethyl-*N,N*,β-trimethyl-3-(phenylmethoxy)benzenepropanamine, characterized by the following 2-theta degrees ±0.2 (Table 1, Figure 1):

| No. | Pos. [°2Th.] | Rel. Int. [%] |
|---|---|---|
| 1 | 5 | 49 |
| 2 | 10 | 20 |
| 3 | 15.1 | 100 |
| 4 | 16.7 | 10 |
| 5 | 19.2 | 4 |
| 6 | 20.2 | 5 |
| 7 | 20.5 | 4 |
| 8 | 21.4 | 5 |
| 9 | 22.3 | 7 |
| 10 | 26.1 | 4 |

X-ray powder diffraction patterns were obtained by Phillips PW3040/60 X'Pert PRO diffractometer; CuK_{α} radiation 1.541874 Ǻ.

This polymorphic form is also referred to herein as Form I.

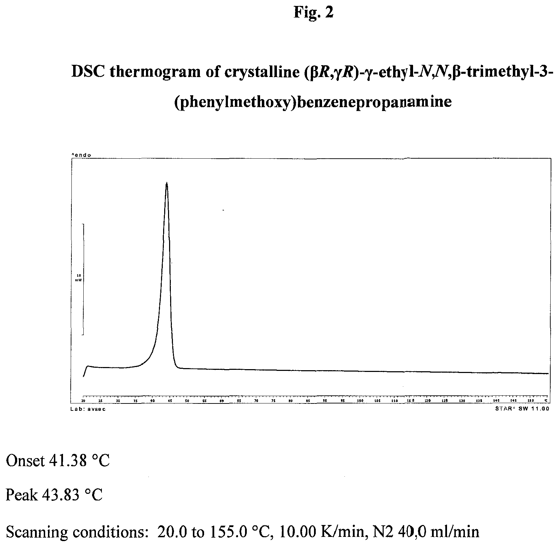

Crystalline (β*R*,γ*R*)-γ-ethyl-*N,N*,β-trimethyl-3-(phenylmethoxy)benzenepropanamine (Form I) can be characterized by the following 2-theta degrees: 5.0, 10.0, 15.1 and 16.7±0.2, and can be further characterized by the following 2-theta degrees: 19.2, 20.2, 20.5, 21.4, 22.3, and 26.1 ±0.2; and can be additionally or alternatively also characterized by the DSC curve shown in Fig. 2.

DSC curves were recorded on DSC1 STAR^{e} System Mettler Toledo. Samples of approx. 3 mg were scanned at a heating rate of 10 °C/min under nitrogen atmosphere in aluminium DSC pans with aluminium perforated lids.

The invention is explained in more detail in the following working examples. The examples, which illustrate improvement in the method according to the invention, have a purely illustrative character and do not limit the extent of the invention in any respect.

### EXAMPLES

### Example 1

### Synthesis of (αR,βR)-β-ethyl-N,N,α-trimethyl-3-(phenylmethoxy)benzenepropanamide

19.36 g of (α*R*,β*R*)-β-ethyl-α-methyl-3-(phenylmethoxy)benzenepropanoic acid were dissolved in 60 mL of toluene. 5.7 mL of thionyl chloride were added dropwise while stirring under a nitrogen atmosphere. Next, 0.21 mL of DMF were added and the mixture was stirred at 30 - 35 °C for about 1.5 h.

A mixture of 31.23 g of potassium carbonate, 13.16 g of dimethylamine hydrochloride and 80 mL of toluene was partially evaporated under reduced pressure. Next, 40 mL of fresh toluene were added and the solvent was again partially evaporated under reduced pressure.

The first reaction mixture was added dropwise to the mixture of potassium carbonate and dimethylamine hydrochloride in toluene over a period of 38 min while stirring under a nitrogen atmosphere at 15 - 30 °C. The resulting mixture was stirred at 50 - 55 °C for about 2 h. Next, the mixture was cooled to about 15 - 20 °C and 80 mL of H₂O were added. The phases were separated and the aqueous phase was washed with 27 mL of toluene. The combined organic phases were washed with 27 mL of 2 M HCl and 27 mL of brine, and dried over anhydrous Na₂SO₄.

In order to remove (α*R*,β*R*)-β-ethyl-α-methyl-3-(phenylmethoxy)-benzenepropanoic acid, which was shown by HPLC analysis to still be present in the final toluene solution, the toluene solution that was being dried over anhydrous Na₂SO₄ was filtered and diluted with toluene to 150 mL. The resulting solution was washed with 45 mL of 5 % potassium carbonate solution and with 45 mL of brine, dried over anhydrous Na₂SO₄, filtered and partially evaporated under reduced pressure. The resulting solution, containing (α*R*,β*R*)-β-ethyl-*N*,*N*,α-trimethyl-3-(phenylmethoxy)benzenepropanamide, was diluted with toluene to 52 mL.
HPLC purity 98.5 area %.

### Example 2

### Synthesis of (βR,γR)-γ-ethyl-N,N,β-trimethyl-3-(phenylmethoxy)benzenepropanamine

36.53 g of a 70 % solution of sodium bis(2-methoxyethoxy)aluminium dihydride (Red-Al) in toluene were added dropwise over a period of 15 min to 51 mL of the (α*R*,β*R*)-β-ethyl-*N,N*,α-trimethyl-3-(phenylmethoxy)benzenepropanamide solution in toluene, prepared in Example 1, while stirring under a nitrogen atmosphere at about 20 - 25 °C. After the addition was completed, the resulting mixture was stirred at about 40 °C under a nitrogen atmosphere for about 2 h. Next, the mixture was added dropwise to 32 mL of 2 M NaOH over a period of about 1 h while stirring at or slightly below 10 °C. The phases were separated and the organic phase was washed with 5 x 51 mL of H₂O. To the organic phase, 1.27 g of activated charcoal were added, stirred for 30 min and filtered off. The filtrate was evaporated under reduced pressure to afford 16.20 g of crude (β*R*,γ*R*)-γ-ethyl-*N,N*,β-trimethyl-3-(phenylmethoxy)benzenepropanamine.
HPLC purity 98.3 area %
HPLC assay: 80 wt. %

### Example 3

### Crystallization of (βR,γR)-γ-ethyl-N,N,β-trimethyl-3-(phenylmethoxy)benzenepropanamine in free base form (Form I)

0.70 g of (β*R*,γ*R*)-γ-ethyl-*N,N*,β-trimethyl-3-(phenylmethoxy)benzenepropanamine (in free base form, HPLC purity 99.1 area %) and 0.70 mL of methanol were added into glass vessel-1, equipped with a magnetic stirrer and sealed with a plastic cover at room temperature, and the mixture was stirred until clear solution-1 was formed. Separately, into glass vessel-2 (equipped with a mechanical stirrer and sealed with a plastic cover) 7.0 mL of purified water were added and cooled to the temperature of 0 °C. The prepared solution-1 was added dropwise to vessel-2 over the next 5 min. After solution-1 was added, the mixture was further stirred for the next 90 min at a temperature of 0 °C of the cooling bath. The prepared suspension was then filtered under dry nitrogen - using a pressure filter - and washed with 1 mL of fresh, purified water. The obtained product was dried using a vacuum plate-dryer at a temperature of 40 °C of the medium and a pressure < 50 mbar for the next 20 h.
Yield: 0.55 g (HPLC purity 99.3 area %)

### Example 4

### Crystallization of (βR,γR)-γ-ethyl-N,N,β-trimethyl-3-(phenylmethoxy)benzenepropanamine in free base form (Form I)

0.70 g of (β*R*,γ*R*)-γ-ethyl-*N,N*,β-trimethyl-3-(phenylmetboxy)benzenepropanamine (in free base form, HPLC purity 99.1 area %) and 0.70 mL of methanol were added into glass vessel-1, equipped with a magnetic stirrer and sealed with a plastic cover at room temperature, and the mixture was stirred until clear solution-1 was formed. Separately, into glass vessel-2 (equipped with a mechanical stirrer and sealed with a plastic cover) 7.0 mL of purified water were added and heated to the temperature of 50 °C. The prepared solution-1 was added dropwise to vessel-2 over the next 7 min. After solution-1 was added, the mixture was further stirred for the next 15 h at a temperature of 50 °C of the heating bath. Furthermore, the mixture was then cooled using the temperature of the heating bath at 40 °C and stirred at these conditions for the next 3 h. The prepared suspension was then filtered under dry nitrogen - using a pressure filter - and washed with 2 mL of fresh, purified water. The obtained product was dried using a vacuum plate-dryer at a temperature of 40 °C of the medium and a pressure < 50 mbar for the next 20 h.
Yield: 0.42 g (HPLC purity 99.1 area %)

### Example 5

### Crystallization of (βR,γR)-γ-ethyl-N,N,β-trimethyl-3-(phenylmethoxy)benzenepropanamine in free base form (Form I)

0.70 g of (β*R*,γ*R*)-γ-ethyl-*N,N*,β-trimethyl-3-(phenylmethoxy)benzenepropanamine (in free base form, HPLC purity 99.1 area %) and 0.70 mL of acetonitrile were added into glass vessel-1, equipped with a magnetic stirrer and sealed with a plastic cover at room temperature, and the mixture was stirred until clear solution-1 was formed. Separately, into glass vessel-2 (equipped with a mechanical stirrer and sealed with a plastic cover) 7.0 mL of purified water were added and cooled to the temperature of 0 °C. The prepared solution-1 was added dropwise to vessel-2 over the next 5 min. After solution-1 was added, the mixture was further stirred for the next 60 min at a temperature of 0 °C of the cooling bath. The prepared suspension was then filtered under dry nitrogen - using a pressure filter - and washed with 1 mL of fresh, purified water. The obtained product was dried using a vacuum plate-dryer at a temperature of 40 °C of the medium and a pressure < 50 mbar for the next 20 h.
Yield: 0.58 g (HPLC purity 99.2 area %)

### Example 6

### Crystallization of (βR,γR)-γ-ethyl-N,N,β-trimethyl-3-(phenylmethoxy)benzenepropanamine in free base form (Form I)

1.00 g of (β*R*,γ*R*)-γ-ethyl-*N,N*,β-trimethyl-3-(phenylmethoxy)benzenepropanamine (in free base form, HPLC purity 98.3 area %, HPLC assay 80 wt. %, from Example 2) and 0.90 mL of methanol were added into glass vessel-1, equipped with a magnetic stirrer and sealed with a plastic cover at room temperature, and the mixture was stirred until clear solution-1 was formed. Separately, into glass vessel-2 (equipped with a mechanical stirrer and sealed with a plastic cover) 9.0 mL of purified water were added and cooled to the temperature of 0 °C and 10 mg of seeding crystals were added (material from Example 5). The prepared solution-1 was then added gradually dropwise to vessel-2 over the next 9 min. After solution-1 was added, the mixture was further stirred for the next 90 min at a temperature of 0 °C of the cooling bath. The prepared suspension was then filtered under dry nitrogen - using a pressure filter - and washed with 1 mL of fresh, purified water. The obtained product was dried using a vacuum plate-dryer at a temperature of 40 °C of the medium and a pressure < 50 mbar for the next 20 h.
Yield: 0.81 g (HPLC purity 98.4 area %, HPLC assay 95.1 wt. %)

### Example 7

### Crystallization of (βR,γR)-γ-ethyl-N,N,β-trimethyl-3-(phenylmethoxy)benzenepropanamine in free base form (Form I)

5.00 g of (β*R*,γ*R*)-γ-ethyl-*N,N*,β-trimethyl-3-(phenylmethoxy)benzenepropanamine (in free base form, HPLC purity 98.3 area %, HPLC assay 80 wt. %, from Example 2) and 4.50 mL of methanol were added into glass vessel-1, equipped with a magnetic stirrer and sealed with a plastic cover at room temperature, and the mixture was stirred (300 rpm) until clear solution-1 was formed. Separately, into a glass reactor (equipped with a pitch-blade mechanical stirrer and sealed) 45.0 mL of purified water were added and cooled to the temperature of 0 °C and 50 mg of seeding crystals (material from Example 6) were added. The prepared solution-1 was then added dropwise to vessel-2 over the next 16 min. After solution-1 was added, the mixture was further stirred for the next 90 min at a temperature of -2 °C of the cooling bath. The prepared suspension was then filtered under dry nitrogen - using a pressure filter - and washed with 1 mL of fresh, purified water. The obtained product was dried using a vacuum plate-dryer at a temperature of 40 °C of the medium and a pressure < 50 mbar for the next 20 h.
Yield: 4.22 g (HPLC purity 98.6 area %, Karl Fischer titration of water 0.09 % content)

### Example 8

### Synthesis of (αR,βR)-β-ethyl-N,N,α-trimethyl-3-(phenylmethoxy)benzenepropanamide

4.86 g of (α*R*,β*R*)-β-ethyl-α-methyl-3-(phenylmethoxy)benzenepropanoic acid were dissolved in 15 mL of toluene. 1.5 mL of thionyl chloride were added dropwise while stirring under a nitrogen atmosphere. Next, 0.05 mL of DMF were added and the mixture was stirred at about 30 °C for about 70 min.

The reaction mixture was added dropwise to a mixture of 7.81 g of potassium carbonate, 3.29 g of dimethylamine hydrochloride and 10 mL of toluene over a period of 14 min while stirring under a nitrogen atmosphere at about 20 °C. The resulting mixture was stirred at 50 - 55 °C for about 2 h. Next, the mixture was cooled to about 15 - 20 °C and 20 mL of H₂O were added. The phases were separated and the aqueous phase was washed with 7 mL of toluene. The combined organic phases were washed with 7 mL of 2 M HCl and 7 mL of brine, and dried over anhydrous Na₂SO₄.

The mixture was filtered and partially evaporated under reduced pressure. The resulting solution was further diluted with toluene to a volume of 13.5 mL. HPLC purity 99.0 area %.

### Example 9

### Synthesis of (βR,γR)-γ-ethyl-N,N,β-trimethyl-3-(phenylmethoxy)benzenepropanamine

8.9 g of a 70 % solution of sodium bis(2-methoxyethoxy)aluminium dihydride (Red-Al) in toluene were added dropwise over a period of 19 min to 13 mL of the (α*R*,β*R*)-β-ethyl-*N,N,α*-trimethyl-3-(phenylmethoxy)benzenepropanamide solution in toluene, prepared in Example 8, while stirring under a nitrogen atmosphere at about 20 °C. After the addition was completed, the resulting mixture was stirred at about 40 °C under a nitrogen atmosphere for about 2 h. Next, the mixture was added dropwise to 8.1 mL of 2 M NaOH over a period of 22 min while stirring slightly below 10 °C. The phases were separated and the organic phase was washed with 7 x 13 mL of H₂O. To the organic phase, 0.31 g of activated charcoal were added, stirred for 30 min and filtered off. The filtrate was evaporated under reduced pressure to afford 2.95 g of crude (β*R*,γ*R*)-γ-ethyl-*N,N*,β-trimethyl-3-(phenylmethoxy)benzenepropanamine.

HPLC purity 99.2 area %
HPLC assay: 96 wt. %).

### Example 10

### Crystallization of (βR,γR)-γ-ethyl-N,N,β-trimethyl-3-(phenylmethoxy)benzenepropanamine in free base form (Form I)

1.30 g of (β*R*,γ*R*)-γ-ethyl-*N,N*,β-trimethyl-3-(phenylmethoxy)benzenepropanamine (in free base form, HPLC purity 99.2 area %, HPLC assay 96 wt. %, from Example 9) and 1.30 mL of acetonitrile were added into glass vessel-1, equipped with a magnetic stirrer and sealed with a plastic cover at room temperature, and the mixture was stirred (300 rpm) until clear solution-1 was formed. Separately, into a glass reactor (equipped with a pitch-blade mechanical stirrer and sealed) 13.0 mL of purified water were added and cooled to the temperature of 0 °C. The prepared solution-1, cooled to 0 - 5 °C, was then added dropwise to vessel-2 over the next 5 min. After the addition of solution-1, the mixture was further stirred for the next 90 min at a temperature of 0 °C of the cooling bath. The prepared suspension was then filtered under dry nitrogen - using a pressure filter - and washed with 2.5 mL of fresh, purified water. The obtained product was dried using a vacuum plate-dryer at a temperature of 40 °C of the medium and a pressure < 50 mbar for the next 20 h.
Yield: 1.09 g (HPLC purity 99.5 area %, Karl Fischer titration of water 0.12 % content)

### Example 11

### Crystallization of (βR,γR)-γ-ethyl-N,N,β-trimethyl-3-(phenylmethoxy)benzenepropanamine in free base form (Form I)

0.90 g of (β*R*,γ*R*)-γ-ethyl-*N,N*,β-trimethyl-3-(phenylmethoxy)benzenepropanamine (in free base form, HPLC purity 98.9 area %) and 5 mL of dichloromethane were added into a glass vessel, equipped with a magnetic stirrer and sealed with a condenser, heated to 40 °C and stirred until a clear solution was formed. After that, dichloromethane was evaporated under reduced pressure at the temperature of 40 °C of the water bath. To the formed oily residue 3 mL of 1-ethyl-3-methylimidazolium ethyl sulfate (30 - 35 °C) were added and the mixture was stirred until a homogeneous suspension was formed. The formed suspension was then cooled to 15 - 20 °C and further stirred for the next 120 min. The product formed was separated from suspension using vacuum filtration under nitrogen flow. The obtained product was dried using a vacuum plate-dryer at a temperature of 25 - 28 °C of the medium and a pressure < 50 mbar for the next 20 h.
Yield: 0.49 g (HPLC purity area 99.7 %)

### Example 12

### Crystallization of (βR,γR)-γ-ethyl-N,N,β-trimethyl-3-(phenylmethoxy)benzenepropanamine in free base form (Form I)

0.90 g of (β*R*,γ*R*)-γ-ethyl-*N,N*,β-trimethyl-3-(phenylmethoxy)benzenepropanamine (in free base form, HPLC purity 98.9 area %) and 5 mL of dichloromethane were added into a glass vessel, equipped with a magnetic stirrer and sealed with a condenser, heated to 40 °C and stirred until a clear solution was formed. After that, dichloromethane was evaporated under reduced pressure at the temperature of 40 °C of the water bath. To the formed oily residue 3 mL of a mixture comprising ethylene glycol (ethane-1,2-diol) and polydimethylsiloxane (Oil KT 3 - drum (2001 - 180 kg, GE Silicones, Waterford) (30 - 35 °C) were added and the mixture was stirred until a suspension was formed. The formed suspension was then cooled to 15 - 20 °C and further stirred for the next 120 min. The prepared suspension was then filtered under dry nitrogen using a pressure filter. The product was dried using a vacuum plate-dryer at a temperature of 25 - 28 °C of the medium and a pressure < 50 mbar for the next 20 h.
Yield: 0.59 g (HPLC purity area 99.4 %)

### Example 13

### Crystallization of (βR,γR)-γ-ethyl-N,N,β-trimethyl-3-(phenylmethoxy)benzenepropanamine in free base form (Form I)

0.90 g of (β*R*,γ*R*)-γ-ethyl-*N,N*,β-trimethyl-3-(phenylmethoxy)benzenepropanamine (in free base form, HPLC purity 98.9 area %) and 5 mL of dichloromethane were added into a glass vessel, equipped with a magnetic stirrer and sealed with a condenser, heated to 40 °C and stirred until a clear solution was formed. After that, dichloromethane was evaporated under reduced pressure at the temperature of 40 °C of the water bath. To the formed oily residue 3 mL of 1-butyl-3 -methylimidazolium tetrafluoroborate (30 - 35 °C) were added and the mixture was stirred until a homogeneous suspension was formed. The formed suspension was then cooled to 15 - 20 °C and further stirred for the next 180 min. The prepared suspension was then filtered under dry nitrogen using a pressure filter. The product was dried using a vacuum plate-dryer at a temperature of 25 - 28 °C of the medium and a pressure < 50 mbar for the next 20 h.
Yield: 0.94 g (HPLC purity area 99.6 %)

### Example 14

### Crystallization of (βR,γR)-γ-ethyl-N,N,β-trimethyl-3-(phenylmethoxy)benzenepropanamine in free base form (Form I)

0.90 g of (β*R*,γ*R*)-γ-ethyl-*N,N*,β-trimethyl-3-(phenylmethoxy)benzenepropanamine (in free base form, HPLC purity 98.9 area %) and 5 mL of dichloromethane were added into a glass vessel, equipped with a magnetic stirrer and sealed with a condenser, heated to 40 °C and stirred until a clear solution was formed. After that, dichloromethane was evaporated under reduced pressure at the temperature of 40 °C of the water bath. To the formed oily residue 3 mL of 1-ethyl-3-methylimidazolium methyl sulfate (30 - 35 °C) were added and the mixture was stirred for 30 min at a bath temperature of 30 °C. After that, the mixture was cooled to 15 - 20 °C and stirred until a homogeneous suspension was formed. The formed suspension was then stirred for the next 150 min. The product formed was separated from suspension using vacuum filtration under nitrogen flow. The product was dried using a vacuum plate-dryer at a temperature of 25 - 28 °C of the medium and a pressure < 50 mbar for the next 20 h.
Yield: 0.88 g (HPLC purity area 99.8 %)

### Example 15

### Crystallization of (βR,γR)-γ-ethyl-N,N,β-trimethyl-3-(phenylmethoxy)benzenepropanamine in free base form (Form I)

1.0 g of (β*R*,γ*R*)-γ-ethyl-*N,N*,β-trimethyl-3-(phenylmethoxy)benzenepropanamine (in free base form, HPLC purity area 96.9 %, HPLC assay 90.9 wt. %) and 3 mL of a mixture comprising ethylene glycol and polydimethylsiloxane (Oil KT 3 - drum (2001 - 180 kg, GE Silicones, Waterford) were added into a glass vessel, equipped with a magnetic stirrer and sealed with condenser, heated to 65 °C and stirred until a clear solution was formed. After that, the mixture was cooled to 15 - 20 °C and stirred until a homogeneous suspension was formed. The formed suspension was stirred for the next 150 min at the end temperature. The product was separated from suspension using vacuum filtration under nitrogen flow and dried using a vacuum plate-dryer at a temperature of 25 - 28 °C of the medium and a pressure < 50 mbar for the next 20 h.
Yield: 0.72 g (HPLC purity area 97.9%)

### Example 16

### Crystallization of (βR,γR)-γ-ethyl-N,N,β-trimethyl-3-(phenylmethoxy)benzenepropanamine in free base form (Form I)

1.0 g of (β*R*,γ*R*)-γ-ethyl-*N,N*,β-trimethyl-3-(phenylmethoxy)benzenepropanamine (in free base form, HPLC purity 96.9 area %, HPLC assay 90.9 wt. %) and 5 mL of dichloromethane were added into a glass vessel, equipped with a magnetic stirrer and sealed with a condenser, heated to 40 °C and stirred until a clear solution was formed. After that, dichloromethane was evaporated under reduced pressure at the temperature of 40 °C of the water bath. The oily residue was then cooled to 20 - 25 °C and 3 mL of a mixture comprising ethylene glycol (ethane-1,2-diol) and polydimethylsiloxane (Oil KT 3 - drum (2001 - 180 kg, GE Silicones, Waterford) (RT (room temperature)) was added. After that, a suspension was formed and stirred for the next 180 min. The product formed was separated from suspension using vacuum filtration under nitrogen flow. The product was dried using a vacuum plate-dryer at a temperature of 25 - 28 °C of the medium and a pressure < 50 mbar for the next 20 h.
Yield: 0.73 g (HPLC purity area 97.8 %)

### Example 17

### Crystallization of (βR,γR)-γ-ethyl-N,N,β-trimethyl-3-(phenylmethoxy)benzenepropanamine in free base form (Form I)

1.0 g of (β*R*,γ*R*)-γ-ethyl-*N,N*,β-trimethyl-3-(phenylmethoxy)benzenepropanamine (in free base form, HPLC purity 96.9 area %, HPLC assay 90.9 wt. %) and 5 mL of dichloromethane were added into a glass vessel, equipped with a magnetic stirrer and sealed with a condenser, heated to 40 °C and stirred until a clear solution was formed. After that, dichloromethane was evaporated under reduced pressure at the temperature of 40 °C of the water bath. To the formed oily residue 3 mL of propylene glycol (30 - 35 °C) were added and the mixture was stirred for 30 min at a bath temperature of 30 °C. After that, the mixture was cooled to 15 - 20 °C and stirred until a homogeneous suspension was formed. The formed suspension was then stirred for the next 60 min. The product formed was separated from suspension using vacuum filtration under nitrogen flow. The product was dried using a vacuum plate-dryer at a temperature of 25 - 28 °C of the medium and a pressure < 50 mbar for the next 20 h.
Yield: 0.44 g (HPLC purity area 99.4 %)

### Example 18

### Crystallization of (βR,γR)-γ-ethyl-N,N,β-trimethyl-3-(phenylmethoxy)benzenepropanamine in free base form (Form I)

1.0 g of (β*R*,γ*R*)-γ-ethyl-*N,N*,β-trimethyl-3-(phenylmethoxy)benzenepropanamine (in free base form, HPLC purity 96.9 area %, HPLC assay 90.9 wt. %) and 5 mL of dichloromethane were added into a glass vessel, equipped with a magnetic stirrer and sealed with a condenser, heated to 40 °C and stirred until a clear solution was formed. After that, dichloromethane was evaporated under reduced pressure at the temperature of 40 °C of the water bath. To the formed oily residue 3 mL of propylene glycol (30 - 35 °C) were added and the mixture was cooled to 15 - 20 °C and stirred until a homogeneous suspension was formed. The formed suspension was then stirred for the next 180 min. The product was separated from suspension using vacuum filtration under nitrogen flow and dried using a vacuum plate-dryer at a temperature of 25 - 28 °C of the medium and a pressure < 50 mbar for the next 20 h.
Yield: 0.65 g (HPLC purity area 99.3 %)

### Example 19

### Crystallization of (βR,γR)-γ-ethyl-N,N,β-trimethyl-3-(phenylmethoxy)benzenepropanamine in free base form (Form I)

9.0 g of (β*R*,γ*R*)-γ-ethyl-*N,N*,β-trimethyl-3-(phenylmethoxy)benzenepropanamine (in free base form, HPLC purity 96.9 area %, HPLC assay 90.9 wt. %) and 30 mL of dichloromethane were added into a glass vessel, equipped with a magnetic stirrer and sealed with a condenser, heated to 40 °C and stirred until a clear solution was formed. After that, dichloromethane was evaporated under reduced pressure at the temperature of 40 °C of the water bath. To the formed oily residue 18 mL of propylene glycol (30 °C) were added and stirred until a homogeneous suspension was formed. After that, the mixture was cooled to 15 °C in 1 h and stirred for the next 60 min. The formed product was separated from suspension using vacuum filtration under nitrogen flow. The product was dried using a vacuum plate-dryer at a temperature of 25 - 28 °C of the medium and a pressure < 50 mbar for the next 72 h.
Yield: 8.1 g (HPLC purity area 99.1 %)

### Example 20

### Crystallization of (βR,γR)-γ-ethyl-N,N,β-trimethyl-3-(phenylmethoxy)benzenepropanamine in free base form (Form I)

9.0 g of (β*R*,γ*R*)-γ-ethyl-*N,N*,β-trimethyl-3-(phenylmethoxy)benzenepropanamine (in free base form, HPLC purity 96.9 area %) and 30 mL of dichloromethane were added into a glass vessel, equipped with a magnetic stirrer and sealed with a condenser, heated to 40 °C and stirred until a clear solution was formed. After that, dichloromethane was evaporated under reduced pressure at the temperature of 40 °C of the water bath. To the formed oily residue 18 mL of a mixture comprising ethylene glycol (ethane-1,2-diol) and polydimethylsiloxane (Oil KT 3 - drum (2001 - 180 kg, GE Silicones, Waterford) (30 °C) were added and stirred until a suspension was formed. After that, the mixture was cooled to 20 °C in 1 h and stirred for the next 60 min. The formed product was separated from suspension using vacuum filtration under nitrogen flow. The product was dried using a vacuum plate-dryer at a temperature of 25 - 28 °C of the medium and a pressure < 50 mbar for the next 72 h.
Yield: 8.2 g (HPLC purity area 97.9 %)

## Claims

1. Crystalline (β*R*,γ*R*)-γ-ethyl-*N,N*,β-trimethyl-3-(phenylmethoxy)benzenepropanamine.

2. Crystalline (β*R*,γ*R*)-γ-ethyl-*N,N*,β-trimethyl-3-(phenylmethoxy)benzenepropanamine according to claim 1, **characterized by** the following 2-theta degrees: 5, 10, 15.1 and 16.7±0.2 in a X-ray powder diffraction pattern measured with CuKα radiation having a wavelength of 0.1541874 nm.

3. Crystalline (β*R*,γ*R*)-γ-ethyl-*N,N*,β-trimethyl-3-(phenylmethoxy)benzenepropanamine according to claims 1 to 2, further **characterized by** the following 2-theta degrees: 19.2, 20.2, 20.5, 21.4, 22.3, and 26.1 ±0.2 in a X-ray powder diffraction pattern measured with CuKα radiation having a wavelength of 0.1541874 nm.

4. Crystalline (β*R*,γ*R*)-γ-ethyl-*N,N*,β-trimethyl-3-(phenylmethoxy)benzenepropanamine according to claim 1 to 3, **characterized by** a DSC curve showing a peak at 43.83 °C with an onset at 41.38 °C under the scanning conditions 20.0 to 155 °C, 10.00 K/min, N₂ 40.0 ml/min.

5. A process for the preparation of crystalline (β*R*,γ*R*)-γ-ethyl-*N,N*,β-trimethyl-3-(phenylmethoxy)benzenepropanamine comprising the following steps:
(a) providing a mixture of (β*R*,γ*R*)-γ-ethyl-*N,N*,β-trimethyl-3-(phenylmethoxy)benzenepropanamine in an organic solvent or mixture thereof;
(b) optionally, treating the mixture of step (a) with an appropriate antisolvent or mixture of antisolvents, and/or cooling,
(c) isolating material with filtration or centrifugation
(d) optionally drying the isolated material
(e) optionally storing the dry, solid material (β*R*,γ*R*)-γ-ethyl-*N,N*,β-trimethyl-3-(phenylmethoxy)benzenepropanamine for more than 24 h.

6. The process according to claim 5, which is a process for the preparation of (β*R*,γ*R*)-γ-ethyl-*N,N*,β-trimethyl-3-(phenylmethoxy)benzenepropanamine and for the subsequent preparation of tapentadol or a pharmaceutically acceptable salt thereof, which further comprises:
(f) preparing tapentadol or a pharmaceutically acceptable salt thereof from the material isolated with filtration or centrifugation, or from the dried isolated material or from the stored solid material (β*R*,γ*R*)-γ-ethyl-*N,N*,β-trimethyl-3-(phenylmethoxy)benzenepropanamine.

7. The process according to any one of claims 5 and 6, wherein step (b) is:
treating the mixture of step (a) with an appropriate antisolvent or mixture of antisolvents.

8. The process according to any one of claims 5 and 6, wherein step (b) is:
cooling the mixture of step (a).

9. The process according to claim 7, wherein step (b) is:
treating the mixture of step (a) with an appropriate antisolvent or mixture of antisolvents and cooling.

10. Use of crystalline (β*R*,γ*R*)-γ-ethyl-*N,N*,β-trimethyl-3-(phenylmethoxy)benzenepropanamine for the preparation of tapentadol or a pharmaceutically acceptable salt thereof.

11. The use of crystalline (β*R*,γ*R*)-γ-ethyl-*N,N*,β-trimethyl-3-(phenylmethoxy)benzenepropanamine according to claim 10, wherein crystalline (β*R*,γ*R*)-γ-ethyl-*N,N*,β-trimethyl-3-(phenylmethoxy)benzenepropanamine according to any of claims 2 to 4 is used.

## Patentansprüche

1. Kristallines (β*R*,γ*R*)-γ-Ethyl-*N,N*,β-trimethyl-3-(phenylmethoxy)benzenpropanamin.

2. Kristallines (β*R*,γ*R*)-γ-Ethyl-*N,N*,β-trimethyl-3-(phenylmethoxy)benzenpropanamin gemäß Anspruch 1, **gekennzeichnet durch** die folgenden 2-Theta Grade: 5, 10, 15,1 und 16,7 ± 0,2 in einem Röntgenpulverbeugungsmuster, das mit CuKa-Strahlung mit einer Wellenlänge von 0,1541874 nm gemessen wird.

3. Kristallines (β*R*,γ*R*)-γ-Ethyl-*N,N*,β-trimethyl-3-(phenylmethoxy)benzenpropanamin gemäß den Ansprüchen 1 bis 2, ferner **gekennzeichnet durch** die folgenden 2-Theta Grade: 19,2, 20,2, 20,5, 21,4, 22,3 und 26,1 ± 0,2 in einem Röntgenpulverbeugungsmuster, das mit CuKa-Strahlung mit einer Wellenlänge von 0,1541874 nm gemessen wird.

4. Kristallines (β*R*,γ*R*)-γ-Ethyl-*N,N*,β-trimethyl-3-(phenylmethoxy)benzenpropanamin gemäß den Ansprüchen 1 bis 3, **gekennzeichnet durch** eine DSC-Kurve, die einen Peak bei 43,83°C mit einem Einsetzen bei 41,38°C unter den Scanningbedingungen 20,0 bis 155°C, 10,00 K/min, N₂ 40,0 ml/min zeigt.

5. Verfahren zur Herstellung von kristallinem (β*R*,γ*R*)-γ-Ethyl-*N,N*,β-trimethyl-3-(phenylmethoxy)benzenpropanamin, umfassend die folgenden Schritte:
(a) Bereitstellen eines Gemisches von (β*R*,γ*R*)-γ-Ethyl-*N,N*,β-trimethyl-3-(phenylmethoxy)benzenpropanamin in einem organischen Lösungsmittel oder Gemisch davon;
(b) gegebenenfalls Behandeln des Gemisches von Schritt (a) mit einem passenden Antilösungsmittel oder Gemisch von Antilösungsmitteln und/oder Kühlen,
(c) Isolieren von Material mit Filtration oder Zentrifugation,
(d) gegebenenfalls Trocknen des isolierten Materials,
(e) gegebenenfalls Lagern des trockenen, festen Materials (β*R*,γ*R*)-γ-Ethyl-*N,N*,β-trimethyl-3-(phenylmethoxy)benzenpropanamin für länger als 24 h.

6. Verfahren gemäß Anspruch 5, welches ein Verfahren zur Herstellung von (β*R*,γ*R*)-γ-Ethyl-*N,N*,β-trimethyl-3-(phenylmethoxy)benzenpropanamin und zur nachfolgenden Herstellung von Tapentadol oder eines pharmazeutisch verträglichen Salzes davon ist, welches ferner umfasst:
(f) Herstellen von Tapentadol oder eines pharmazeutisch verträglichen Salzes davon aus dem Material, das mit Filtration oder Zentrifugation isoliert wird, oder aus dem getrockneten isolierten Material oder aus dem gelagerten festen Material (β*R*,γ*R*)-γ-Ethyl-*N,N*,β-(phenylmethoxy)benzenpropanamin.

7. Verfahren gemäß einem der Ansprüche 5 und 6, wobei Schritt (b) ist:
Behandeln des Gemisches von Schritt (a) mit einem passenden Antilösungsmittel oder
Gemisch von Antilösungsmitteln.

8. Verfahren gemäß einem der Ansprüche 5 und 6, wobei Schritt (b) ist:
Kühlen des Gemisches von Schritt (a).

9. Verfahren gemäß Anspruch 7, wobei Schritt (b) ist:
Behandeln des Gemisches von Schritt (a) mit einem passenden Antilösungsmittel oder
Gemisch von Antilösungsmitteln und Kühlen.

10. Verwendung von kristallinem (β*R*,γ*R*)-γ-Ethyl-*N,N,*β-trimethyl-3-(phenylmethoxy)-benzenpropanamin zur Herstellung von Tapentadol oder einem pharmazeutisch verträglichen Salz davon.

11. Verwendung von kristallinem (β*R*,γ*R*)-γ-Ethyl-*N,N,*β-trimethyl-3-(phenylmethoxy)-benzenpropanamin gemäß Anspruch 10, wobei kristallines (β*R*,γ*R*)-γ-Ethyl-*N,N,*β-trimethyl-3-(phenylmethoxy)benzenpropanamin gemäß einem der Ansprüche 2 bis 4 verwendet wird.

## Revendications

1. (β*R*,γ*R*)-γ-éthyl-*N,N,*β-triméthyl-3-(phénylméthoxy)benzènepropanamine cristalline.

2. (β*R*,γ*R*)-γ-éthyl-*N,N,*β-triméthyl-3-(phénylméthoxy)benzènepropanamine cristalline selon la revendication 1, **caractérisée par** les degrés 2-thêta suivants : 5, 10, 15,1 et 16,7 ± 0,2 dans un spectre de diffraction des rayons X sur poudre, mesuré à l'aide d'un rayonnement CuKa possédant une longueur d'onde de 0,1541874 nm.

3. (β*R*,γ*R*)-γ-éthyl-*N,N,*β-triméthyl-3-(phénylméthoxy)benzènepropanamine cristalline selon les revendications 1 à 2, **caractérisée en outre par** les degrés 2-thêta suivants : 19,2, 20,2, 20,5, 21,4, 22,3 et 26,1 ± 0,2 dans un spectre de diffraction des rayons X sur poudre, mesuré à l'aide d'un rayonnement CuKa possédant une longueur d'onde de 0,1541874 nm.

4. (β*R*,γ*R*)-γ-éthyl-*N,N,*β-triméthyl-3-(phénylméthoxy)benzènepropanamine cristalline selon les revendications 1 à 3, **caractérisée par** une courbe DSC présentant un pic à 43,83 °C avec un début à 41,38 °C dans les conditions de balayage 20,0 à 155 °C, 10,00 K/min, N₂ 40,0 ml/min.

5. Un procédé de préparation de (β*R*,γ*R*)-γ-éthyl-*N,N,*β-triméthyl-3-(phénylméthoxy)benzènepropanamine cristalline comprenant les étapes suivantes :
(a) préparation d'un mélange de (β*R*,γ*R*)-γ-éthyl-*N,N,*β-triméthyl-3-(phénylméthoxy)benzènepropanamine dans un solvant organique ou un mélange de solvants organiques ;
(b) facultativement, traitement du mélange de l'étape (a) avec un antisolvant ou un mélange d'antisolvants approprié(s), et/ou refroidissement ;
(c) isolement de la matière par filtration ou centrifugation ;
(d) facultativement, séchage de la matière isolée ;
(e) facultativement, stockage de la matière solide sèche (β*R*,γ*R*)-γ-éthyl-*N,N*,β-triméthyl-3-(phénylméthoxy)benzènepropanamine pendant plus de 24 heures.

6. Le procédé selon la revendication 5, qui est un procédé de préparation de (β*R*,γ*R*)-γ-éthyl-*N,N,*β-triméthyl-3-(phénylméthoxy)benzènepropanamine et de préparation ultérieure de tapentadol ou d'un sel de celui-ci acceptable sur le plan pharmaceutique, qui comprend en outre :
(f) la préparation de tapentadol ou d'un sel de celui-ci acceptable sur le plan pharmaceutique à partir de la matière isolée par filtration ou centrifugation, ou de la matière isolée séchée ou de la matière solide stockée (β*R*,γ*R*)-γ-éthyl-*N,N,*β-triméthyl-3-(phénylméthoxy)benzènepropanamine.

7. Le procédé selon l'une quelconque des revendications 5 et 6, dans lequel l'étape (b) est : traitement du mélange de l'étape (a) par un antisolvant ou un mélange d'antisolvants approprié(s).

8. Le procédé selon l'une quelconque des revendications 5 et 6, dans lequel l'étape (b) est : refroidissement du mélange de l'étape (a).

9. Le procédé selon la revendication 7, dans lequel l'étape (b) est : traitement du mélange de l'étape (a) par un antisolvant ou un mélange d'antisolvants approprié(s) et refroidissement.

10. Utilisation de (β*R*,γ*R*)-γ-éthyl-*N,N,*β-triméthyl-3-(phénylméthoxy)-benzènepropanamine cristalline pour la préparation de tapentadol ou d'un sel de celui-ci acceptable sur le plan pharmaceutique.

11. L'utilisation de (β*R*,γ*R*)-γ-éthyl-*N,N,*β-triméthyl-3-(phénylméthoxy)benzènepropanamine cristalline selon la revendication 10, dans laquelle de la (β*R*,γ*R*)-γ-éthyl-*N,N,*β-triméthyl-3-(phénylméthoxy)benzènepropanamine cristalline selon l'une quelconque des revendications 2 à 4 est utilisée.
